Europäisches Patentamt

European Patent Office

Office européen des brevets

(19)

(11) Veröffentlichungsnummer : **0 229 308 B1**

(12) # EUROPÄISCHE PATENTSCHRIFT

(45) Veröffentlichungstag der Patentschrift :
05.06.91 Patentblatt 91/23

(51) Int. Cl.⁵ : **A61B 6/14**

(21) Anmeldenummer : 86117090.0

(22) Anmeldetag : 08.12.86

(54) **Zahnärztliches Röntgendiagnostikgerät zur Erstellung von Panorama-Schichtaufnahmen vom Kiefer eines Patienten.**

(30) Priorität : 20.12.85 DE 3545436

(43) Veröffentlichungstag der Anmeldung :
22.07.87 Patentblatt 87/30

(45) Bekanntmachung des Hinweises auf die
Patenterteilung :
05.06.91 Patentblatt 91/23

(84) Benannte Vertragsstaaten :
DE FR GB IT

(56) Entgegenhaltungen :
DE-A- 2 461 441
DE-A- 3 125 243
DE-A- 3 321 057
DE-C- 472 413
FR-A- 1 195 082
US-A- 2 434 827
Der Siemens AG, Geschäftsbereich Dental,
Prospekt M-D 80/1361; WS 08832

(73) Patentinhaber : Siemens Aktiengesellschaft
Wittelsbacherplatz 2
W-8000 München 2 (DE)

(72) Erfinder : Döbert, Michael
Zedernstrasse 3
W-6143 Lorsch (DE)
Erfinder : Günther, Werner
Odenwaldstrasse 20
W-6140 Bensheim (DE)
Erfinder : Heubeck, Erich
Blütenweg 11
W-6140 Bensheim (DE)
Erfinder : Müther, Manfred
Adolf-Kolping-Strasse 20
W-6140 Bensheim (DE)
Erfinder : Moliter, Dieter
Kurt-Schumacher-Strasse 9
W-6842 Bürstadt (DE)

EP 0 229 308 B1

Jouve, 18, rue Saint-Denis, 75001 PARIS

**Beschreibung**

Aus dem Prospekt der Siemens AG, Geschäftsbereich Dental, M-D 80/1361 ; WS 08832 mit dem Titel "Mit überlegener Technik zu besseren Ergebnissen....." ist unter der Bezeichnung ORTHOPANTOMOGRAPH 10 ein zahnärztliches Röntgendiagnostikgerät zur Erstellung von Panorama-Schichtaufnahmen vom Kiefer eines Patienten bekannt, welches eine an einem Laufwagen eines Statives angeordnete Dreheinheit enthält. Die Dreheinheit weist einen waagrechten Tragebalken auf, der an seinem einen Ende die Strahlenquelle und diametral dazu an seinem anderen Ende eine mit einer speziellen Mechanik gekuppelte Filmkassettenhalterung aufnimmt. In der Filmkassettenhalterung ist die den Röntgenfilm aufnehmende Filmkassette ortsfest, d.h. nicht verstellbar eingelegt. Die Mechanik ist so ausgelegt, daß die Filmkassettenhalterung relativ zur Strahlenquelle mit einer bestimmten Geschwindigkeit, die mit der Laufgeschwindigkeit der Strahlenquelle im Verlauf der Drehbewegung des Trägers synchronisiert ist, verstellt wird. Strahlenquelle und Filmkassette werden derart zueinander verstellt, daß von der Strahlenquelle ausgehende Strahlen auf die Filmkassette im wesentlichen senkrecht auftreffen.

Die Verstellmechanik, welche erforderlich ist um die Dreheinheit in der dem Zahnbogen entsprechenden Umlaufkurve zu verstellen, ist vergleichsweise kompliziert und hinsichtlich der mechanischen Konstruktion relativ aufwendig, insbesondere um die erforderliche Abstimmung der Bewegungsabläufe von Dreheinheit einerseits und Filmkassettenhalterung andererseits zu erreichen.

Diese Nachteile weist auch eine andere, aus der DE-A-3 125 243 bekannte Röntgenvorrichtung auf, bei der eine Schwenkplatte mit lotrechter Welle vorgesehen ist, auf der der den Röntgenstrahler und den Filmkassettenhalter aufnehmende Schwenkarm drehbar gelagert ist. Die Schwenkplatte wird dabei von Rollen getragen, die sich an der Bodenwand einer Kammer abwälzen. Ein Schwenkglied ist mittels paralleler Arme an einem ortsfesten Träger so gelagert, daß sie eine bogenförmige Bewegung ohne Drehung um die eigene Achse ausführt.

Aus der DE-A-2 461 441 ist eine Vorrichtung und ein Verfahren zur Herstellung von radiografischen Panorama-Aufnahmen bekannt, welches vom Aufbau her ausschließlich für Aufnahmen vom Schädel eines liegenden Patienten vorgesehen ist. Demgemäß ist ein u.a. in der Höhe einstellbarer Patiententisch vorgesehen, auf dem der Patient in Liegestellung positioniert wird. Im Bereich des Schädels ist, ähnlich wie bei einem Computertomographgerät, eine Scheibe vorgesehen, die den Röntgenstrahler und eine Röntgenfilmkassette trägt. Um den Patienten nicht verstellen zu müssen, kann das Drehzentrum der Scheibe verschoben werden. Hierzu ist die Scheibe an einem Kipprahmen drehbar gelagert, der um einen Gelenkzapfen verschwenkt werden kann. Zur Lagerung der Scheibe sind mehrere am Umfang angeordnete und in entsprechende Nuten eingreifende Rollen vorgesehen, wobei ein Rollenpaar über eine Riemenscheibe von einem Elektromotor mit konstanter Drehzahl angetrieben wird. Die Filmkassettenbewegung erfolgt mittels eines separaten Antriebes. Beide Antriebe werden entsprechend einer gewünschten Filmaufnahme gesteuert.

Abgesehen davon, daß das beschriebene Gerät ausschließlich zur Erstellung von radiografischen Panoramaaufnahmen eines liegenden Patienten geeignet ist, dient das Verschwenken des Kipprahmens dazu, den Durchstrahlungsbereiches verändern zu können, um beispielsweise zu vermeiden, daß die Wirbelsäule des Patienten mit abgebildet wird oder daß andere Objektteile, die evtl. Schatten werfen könnten, auf dem Film abgebildet werden. Das Verstellen des Kipprahmens erfolgt nicht während des Umlaufs der Scheibe sondern nur bei abgeschaltetem Strahler.

Aus der DE-A-3 321 057 ist ferner ein Untersuchungsgerät bekannt, bei dem Strahlenquelle und ein dieser zugeordneter Detektor an einem Laufring gehaltert sind, der in einem ortsfesten Stützring gelagert ist. Laufring und Stützring sind hochkant angeordnet, um den Patienten durch die Ringöffnung hindurchfahren zu können. Mit einem solchen Untersuchungsgerät können Transversaldurchstrahlungen zum Zwecke der Erstellung von Schnittbildern quer zur Körperlängsachse erzielt werden.

Der im Anspruch 1 angegebenen Erfindung liegt die Aufgabe zugrunde, ein zahnärztliches Röntgendiagnostikgerät zum Erstellen von Panorama-Schichtaufnahmen vom Kiefer eines Patienten zu schaffen, welches für stehende Patienten geeignet ist und mit welchem sich gegenüber bekannten Geräten dieser Art mittels relativ einfacher und unkomplizierter Mechanik nicht nur die üblichen Standardaufnahmen vom Kiefer eines Patienten erstellen lassen ; mit welchem der Arzt vielmehr noch die Möglichkeit hat, individuell, d.h. an jeder beliebigen Stelle des Kieferbogens und dort in jeder beliebigen Schichtlage Aufnahmen in optimaler Bildqualität, also bei senkrechter Durchstrahlung und unter Vermeidung von Schattenbildungen, machen zu können.

Ein wesentliches Merkmal der Erfindung ist, daß durch die Eigendrehung des Drehringes, durch die Schwenkbewegung des Drehringes quer zur Symmetrieachse des Objekts und durch eine Änderung des Abstandes des Drehringes zum Laufwagen, jede beliebige Ablaufkurve mit optimaler Durchstrahlrichtung gefahren werden kann, was bedeutet, daß neben den Standardaufnahmen in einer Schichtlage jegliche Spezialaufnahmen, wie Sinus- oder Kiefergelenkaufnahmen, in beliebigen Schichtlagen erstellt werden können. Die hierzu vorzusehenden Ablaufkurven sind vergleichsweise einfach ; sie setzen sich zusammen aus der Drehbewegung, die der Drehring um seine Mittelpunktsachse ausführt, aus einer Querbewegung, die der Drehring durch Schwenken um die am Lagerteil angeordnete Schwenkachse ausführt und aus einer Längsbewegung, mit der sich der Abstand des Drehringes zum Laufwagen verändern läßt.

Weitere Vorteile ergeben sich aus den Unteransprüchen und der nachfolgenden Beschreibung eines Ausführungsbeispieles anhand der Zeichnung. Es zeigen :

Figur 1 eine Ausführungsform des erfindungsgemäßen Gerätes in einer schaubildlichen Darstellung,

Figur 2 eine erste Ausführungsform einer Anordnung des Drehringes am Laufwagen mit vereinfachter Darstellung der Verstellmechanik,

Figur 3 den Drehring und dessen Lagerung in einer Seitenanansicht, teilweise im Schnitt,

Figur 4 einen Ausschnitt aus der Figur 3,

Figur 5 eine erste Ausführungsform, betreffend den Aufbau des Drehringes im Schnitt,

Figur 6 eine zweite Ausführungsform eines Drehringes,

Figur 7 eine dritte Ausführungsform eines Drehringes,

Figur 8 eine Prinzipskizze mit Darstellung des Bewegungsablaufes,

Figur 9 eine andere Ausführungsform einer Halterung des in Figur 1 dargestellten Drehringes,

Figur 10 eine vereinfachte Darstellung des Antriebs des Drehringes nach Figur 9.

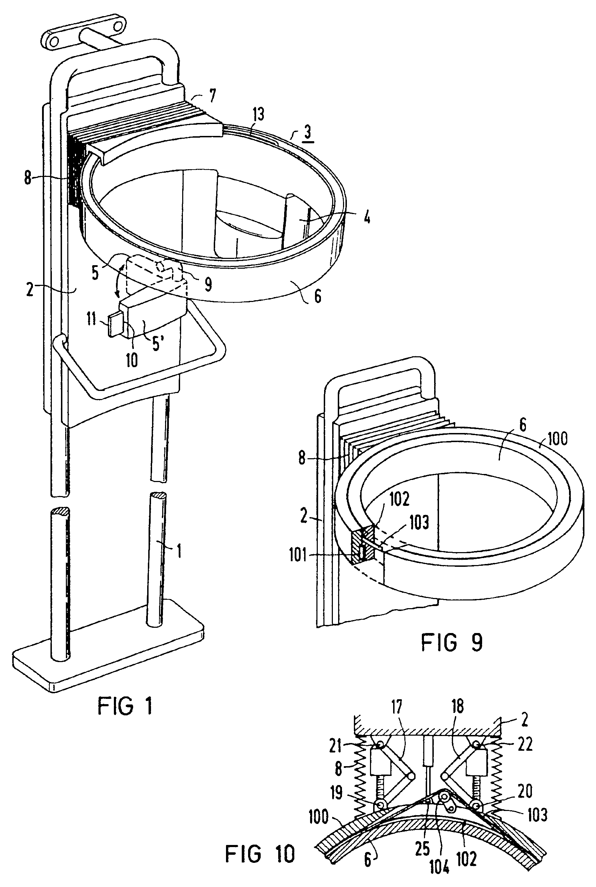

Die Figur 1 zeigt in einer schaubildlichen Darstellung eine Ausführung eines Röntgendiagnostikgerätes nach der Erfindung. Das Gerät enthält ein aus zwei Standrohren gebildetes Stativ 1, an dem ein Laufwagen 2 höhenverstellbar angeordnet ist. Am Laufwagen 2 ist eine allgemein mit 3 bezeichnete Dreheinheit gehalten, die einerseits ein Röntgenstrahlenquelle 4 und diametral dazu eine Filmkassettenhalterung 5 enthält. Träger für Röntgenstrahlenquelle 4 und Filmkassettenhalterung 5 ist ein in sich geschlossener Drehring 6, der in einer später noch näher erläuterten Weise in einem Lagerteil 7 drehund schwenkbar gelagert ist. Die Verstellmechanik zwischen Laufwagen 2 und Drehing 6 ist durch einen Faltenbag 8 abgedeckt.

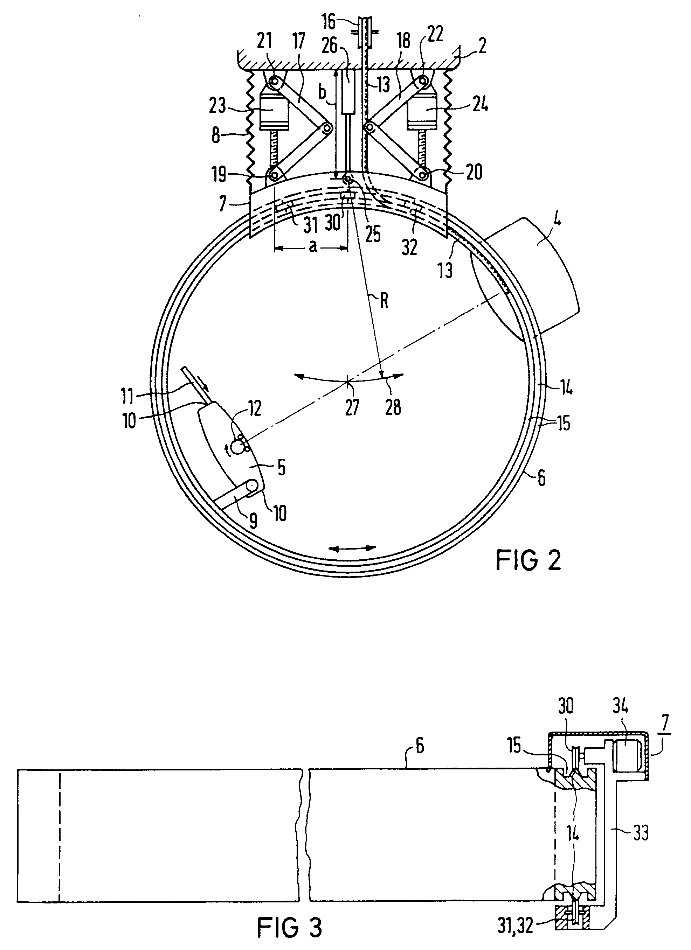

Die Strahlenquelle 4 ist am Drehring 6 fest angeordnet ; die Filmkassettenhalterung 5 dagegen ist am freien Ende eines abgekröpften, am Drehring 6 befestigten Tragarrmes 9 um eine vertikale Achslagerung schwenkbar gehalten. Die Filmkassette kann so zur besseren Patientenpositionierung bzw. für Spezialaufnahmen (Ceph-Aufnahmen) aus der in Figur 1 gestrichelt eingezeichneten Gebrauchsposition in die mit durchgehenden Linien gezeichnete Nichtgebrauchsposition gebracht werden. Die Filmkassettenhalterung 5 enthält an beiden Stirnseiten einen Schlitz 10 ; über den einen wird die mit 11 bezeichnete Filmkassette eingeführt, über den anderen nach der Filmbelichtung ausgefahren. Innerhalb der Filmakassettenhalterung 5 wird der Film, wie in Fig. 2 schematisch dargestellt, mittels geeigneter Transportrollen 12 mit einer festgelegten Geschwindigkeit an der Belichtungsstelle, an der der Zentralstrahl auftrifft, vorbeigeführt.

Die elektrische Versorgung der Strahlenquelle 4 erfolgt mittels einer Leitung 13, die an einer von zwei zu beiden Seiten eines Laufprofils 14 angeordneten Ringnuten 15 bis zum Lagerteil 7 und von dort in den Laufwagen 2 über eine Umlenkrolle 16 od.dgl. bis zu einem nicht dargestellen Versogungsteil geführt ist.

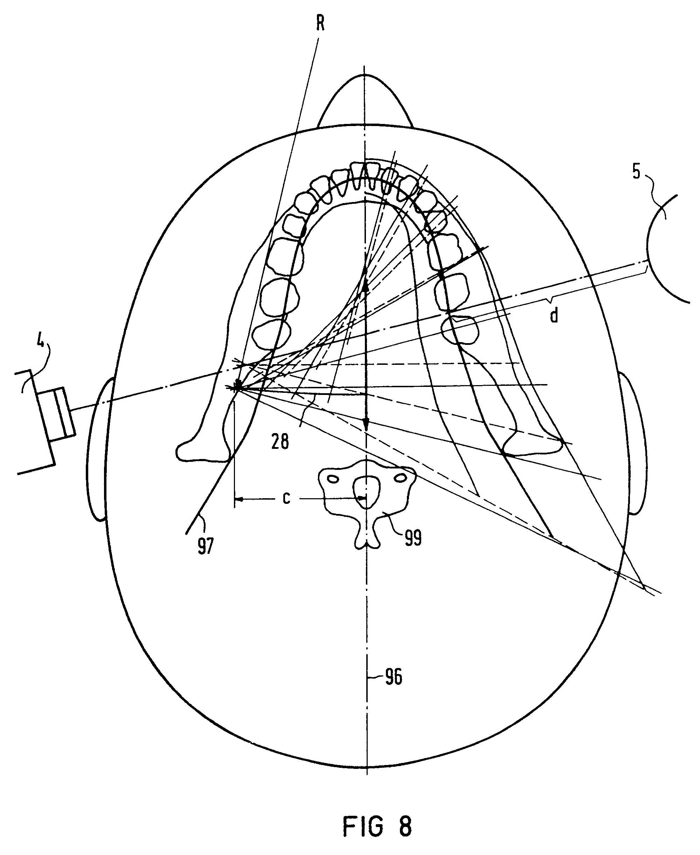

Zur Verstellung des Drehringes 6 sind zwei Scherenarme 17, 18 vorgesehen, deren eine Enden an den mit 19 und 20 bezeichneten Gelenkstellen am Lagerteil 7 und deren andere Enden an den mit 21 und 22 bezeichneten Gelenkstellen am Laufwagen 2 angelenkt sind. Zwischen den Gelenkstellen 19 und 21 einerseits und 20, 22 andererseits sind Spindelantriebe 23, 24 vorgesehen, welche über eine nicht dargestellte Steuereinrichtung individuell angesteuert werden können. Mittig zwischen den beiden Gelenkstellen 19 und 20 (Abstand a) enthält das Lagerteil 7 eine Schwenkachse 25, an der das eine Ende eines Teleskoparmes 26 angelenkt ist, dessen anderes Ende starr am Laufwagen 2 befestigt ist. In Verbindung mit der vorbeschriebenen Scherenarmkonstruktion ist es möglich, bei gleichmäßigem Antrieb beider Spindelantriebe 23, 24 den Drehring 6 parallel zum Laufwagen 2 zu verstellen, also den Abstand b zu verändern, oder bei nicht gleichmäßiger Verstellung der beiden Spindelantriebe 23, 24 den Drehring 6 um die Schwenkachse 25 so zu verschwenken, daß der Mittelpunkt 27 des Drehringes 6 eine Querbewegung (von etwa ± 40 mm) mit dem Radius R (350 mm) in der mit 28 angegebenen Pfeilrichtung ausführt. In Verbindung mit der nachfolgend noch näher erläuterten Eigendrehbewegung, die der Drehring 6 um seine Mittelpunktachse 27 ausführt, kann so der Bewegungsablauf, der bei bekannten Geräten mittels der eingangs erwähnten aufwendigen Mechanik erzielt wird, auf relativ einfache Weise nachvollzogen werden. Die Schwenkachse liegt auf der Symmetrieachse des zu durchstrahlenden Objekts (Kieferbogen) ; der Schwenkradius (R) ist an sich beliebig, jedoch unter Beachtung einer gewählten Anordnung von Strahlenquelle und Filmkassettenhalter so anzulegen, daß sich der in Figur 8 schematisch aufgezeigte Bewegungsablauf in bezug auf das Objekt ergibt.

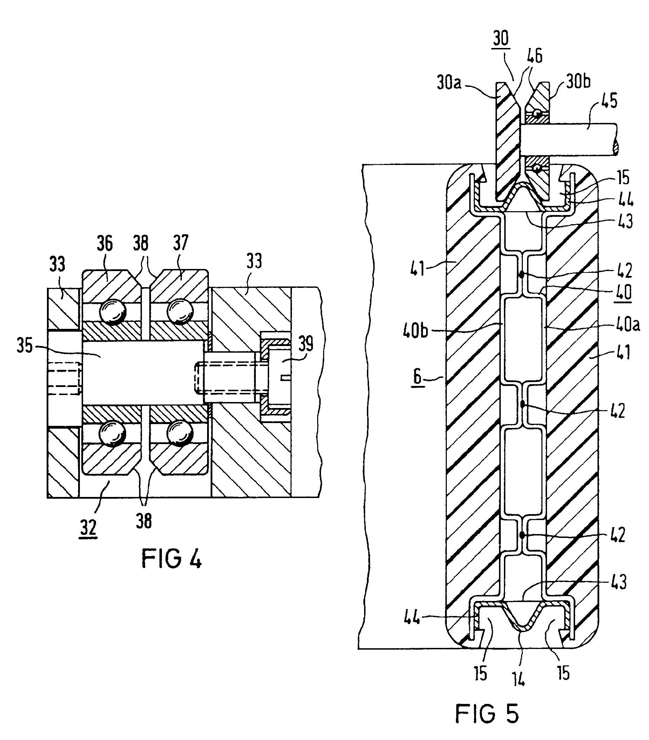

Das Lagerteil 7 dient einerseits zur Halterung des Drehringes 6, andererseits auch dazu, um diesen, wie erwähnt, um seine Mittelpunktachse 27 verdrehen zu können. Zu diesem Zweck ist der Drehring 6 an seiner Oberseite mittels einer Führungsrolle 30 und an seiner Unterseite mittels zweier Führungsrollen 31, 32 gelagert. Die Führungsrollen 30 bis 32 bilden eine Dreieckslagerung mit der Rolle 30 in der Mitte oben und den beiden Rollen 31, 32 zu beiden Selten unten. Hierzu ist der Rahmen 33 des Lagerteils 7 entsprechend winkelig ausgebildet. Die obere Rolle 30 ist als Antriebsrolle ausgebildet und mit einem allgemein mit 34 bezeichneten Antriebsmotor gekuppelt. Die beiden unteren Rollen 31, 32 sind nur Führungsrollen, ihre Achsen sind zur Einstellung des Führungsspliels inne-halb der durch die Rollen 30 bis 32 gegebenen Dreieckslagerung im Rahmen 33 verstellbar angeordnet. Näheres ist aus Figur 4 ersichtlich, anhand der, am Beispiel der Führungsrolle 32, deren Aufbau und Lagerung erläutert wird.

Die Rolle 32 besteht aus zwei nebeneinander auf einer gemeinsamen Achse 35 angeordneten Kugellagern 36, 37, deren Innenringe auf der Achse 35 fest aufgespannt sind und deren Außenringe gegeneinander frei drehbar sind und zum Laufprofil 14 passende Laufflächen 38 aufweisen. Die Achse 35 ist eine Exzenterachse, die im Rahmen 33 in geeigneter Weise, z.B. mittels Innensechskant 39, verstellbar ist, wodurch das Lagerspiel zwischen Antriebsrolle 30 und Führungsrolle 32 eingestellt werden kann. Durch die Exzenterverstellung läßt sich auch der Drehring in bezug auf die Horizontale einjustieren.

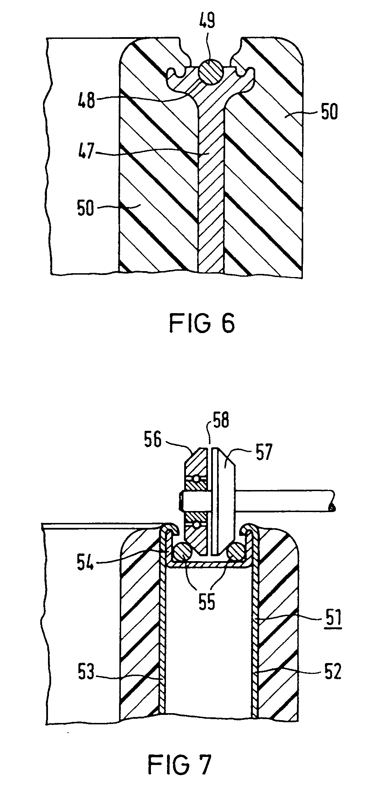

Die Figur 5 zeigt eine erste Ausführungsform betreffend den Aufbau des Drehringes 6. Die Figur zeigt den Drehring im Schnitt, und zwar im Bereich der Antriebsrolle 30. Der Drehring besteht aus einem metallenen Träger 40, der beidseitig mit einem geeigneten Schaumstoff 41 umschäumt ist. Der Träger 40 besteht bei diesem Ausführungsbeispiel aus zwei Hälften 40a, 40b aus jeweils gerolltem Blechband, welche an den mit 42 bezeichneten Stellen miteinander verschweißt sind. Die Hälften 40a, 40b bilden an ihren oberen und unteren Enden eine U-förmige Aufweitung 43, in der ein weiteres gerolltes Profilblech 44 eingeschweißt ist, welches mittig das in Figur 2 mit 14 bezeichnete Laufprofil und beidseitig davon die – über den Umfang des Drehringes gesehen – ringförmigen Nuten 15 bilden, welche, wie erwähnt, auch für die Kabelführung vorgesehen werden können.

Die Antriebsrolle 30 besteht ebenfalls aus zwei Hälften, einer linken Hälfte 30a, die mit der Antriebsachse 45 fest verbunden ist, und einer rechten Hälfte 30b, die nach Art eines Kugellagers ausgebildet ist, dessen Innenring wiederum fest mit der Achse 45 verbunden ist und dessen Außenring zusammen mit der Rollenhälfte 30a Laufflächen 46 für das Laufprofil 14 bilden.

Die Figur 6 zeigt eine zweite Ausführungsform eines Drehringes. Hier ist ein etwa I-förmiger Träger 47 vorgesehen, der oben und unten eine umlaufende Ringnut 48 aufweist, in die ein die Laufflächen für das Laufprofil 14 bildender Drahtring 49 eingelegt ist.

Auch bei dieser Ausführungsform ist der Träger beidseitig mit einem Schaumstoff 50 umschäumt.

Die Figur 7 zeigt eine dritte Ausführungsform eines Drehringes ; bei dieser ist ein ebenfalls mit Schaumstoff umschäumter Träger 51 vorhanden. Dieser besteht jedoch auf zwei beabstandeten Blechteilen 52, 53 und an Ober- und Unterseite eingeschweißten U-förmigen Profilblechen 54, in die zwei Drahtringe 55 eingelegt sind. Die Drahtringe 55 bilden im Gegensatz zu den zuvor beschriebenen Ausführungsformen zwei Laufprofile, an denen die Stütz- und Antriebsrollen (hier eine Antriebsrolle 58 gezeigt) aufliegen. Die Rollen weisen schräge Außenflächen 56, 57 auf, welche sich an den Laufprofilen 55 abwälzen. Die Antriebsrolle 58 ist ansonsten wie die in Figur 5 gezeigte Antriebsrolle 30 aufgebaut. Die Drahtringe können über den gesamten Umfang des Drehringes oder auch nur über den für die Verstellung notwendigen Teil des Umfanges angeordnet sein. Im letzteren Fall ist es zweckmäßig, die beiden Drehringenden durch geeignete Klemmteile im Profilblech 58 zu fixieren.

Alternativ zu der erwähnten Umschäumung der Träger können diese auch durch aufgesetzte Schaumstoffprofile abgedeckt sein.

Wenngleich es zwar fertigungstechnisch vorteilhaft ist, einen in sich geschlossenen Drehring vorzusehen, so ist dies Jedoch nicht zwingend notwendig; es genügt vielmehr, wenn der Ring wenigstens den Winkelbereich, der der dem Zahnbogen entsprechenden Umlaufkurve entspricht, ringförmig ausgebildet ist. Dieser Umfangswinkel beträgt mindestens 240 bis 270°.

Einen geschlossenen Drehring wird man bei einer Ausführung gemäß den Figuren 9 und 10 vorsehen, bei der der Drehring 6 konzentrisch zu einem ringförmigen Lagerteil 100 angeordnet ist. Bei diesem Ausführungsbeispiel ist das ringförmige Lagerteil 100, wie bei dem Lagerteil 7 in der Ausführung nach den Figuren 1 und 2 mittels der bereits beschriebenen Verstellmechanik am Laufwagen 2 gehaltert. Der Drehring 6 ist mittels mehrerer am Umfang angeordneter Lagermittel 101 (Kugeln oder Rollen) gelagert und enthält eine umlaufende Nut 102 in die ein Flachriemen 103 eingelegt ist, der als Endlosriemen um eine Antriebsrolle 104 geführt ist, welche zusammen mit einem nicht näher bezeichneten Antrieb am ortsfesten Lagerteil 100 derart gelagert ist, daß eine ausreichende Riemenspannung unter Vermeidung eines Schlupfes zwischen Riemens und Drehring gegeben ist.

Die Figur 8 zeigt anhand einer Prinzipskizze den Strahlenverlauf für die rechte Kieferhälfte eines Patienten, und zwar mit durchgezogenen Linien mit dem erfindungsgemäßen Gerät und mit unterbrochenen Linien mit einem Gerät nach dem Stand der Technik. In der Darstellung sind mit der Position 4, die Strahlenquelle, mit 5, die Filmkassettenhalterung, mit 28 die Bewegungsbahn des mechanischen Drehpunktes, mit 96 die Symmetrieachse des Objekts, mit 97 der Schichtmittenverlauf und mit 99 die Wirbelsäule des Patienten bezeichnet. Aus der Darstellung ist entnehmbar, daß im Vergleich zum Stand der Technik der mechanische Drehpunkt näher an der Wirbelsäule liegt, wodurch sich zwangsläufig verwischte Abbildung der gegenüberliegenden Kieferhälfte, merklich reduziert.

## Ansprüche

1. Zahnärztliches Röntgendiagnostikgerät zur Erstellung von Panorama-Schichtaufnahmen vom Kiefer eines Patienten, enthaltend eine an einem Laufwagen (2) eines Stativs (1) angeordnete Dreheinheit (3) mit einem Träger (6), an dem eine Strahlenquelle (4) und diametral dazu eine Halterung (5) für eine einen Röntgenfilm aufnehmende Filmkassette (11) derart verstellbar gehalten sind, daß von der Strahlenquelle ausgehende, das Objekt (Kiefer) durchdringende Strahlen auf den Röntgenfilm im wesentlichen senkrecht auftreffen, enthaltend ferner eine erste Verstelleinrichtung (12), mit welcher die Filmkassette (11) relativ zur Strahlenquelle (4) verstellbar ist, und eine zweite Verstelleinrichtung, mit welcher der Träger (6) in einer dem Zahnbogen entsprechenden Umlaufkurve verstellbar ist, wobei die zweite Verstelleinrichtung erste Verstellmittel (30 bis 34), mit welchen der Träger (6) um eine erste vertikale Achse (27) gedreht werden kann, zweite Verstellmittel (17 bis 25), mit welchen der Träger (6) während der Drehbewegung um die erste Achse um eine Schwenkachse (25) eine Schwenkbewegung quer zur Symmetrieachse des Objektes ausführen kann, und dritte Verstellmittel (17 bis 26), mit denen der Abstand (b) des Trägers (6) zum Laufwagen (2) verstellbar ist, enthält, wobei der Schwenkradius (R) und die Auslenkung (c) der Schwenkbewegung, die mit den zweiten Verstellmitteln erzielbar sind, so gewählt sind, daß bei Bewegung der Dreheinheit stets eine senkrechte Durchstrahlrichtung durch das Objekt bei konstantem Abstand (d) von Objekt und Film erzielt wird.

2. Gerät nach Anspruch 1, bei dem der Träger als vorzugsweise geschlossener Drehring (6) ausgebildet ist, welcher an einem mit dem Laufwagen (2) verbundenen, die Schwenkachse (25) aufnehmenden Lagerteil (7, 100) drehbar gehalten ist, wobei der Mittelpunkt (27) des Drehringes (6) die Drehachse für die ersten Verstellmittel (30 bis 34) bildet.

3. Gerät nach Anspruch 2, bei dem das Lagerteil (7, 100) mittels zweier, beidseitig der Schwenkachse (25) in einem Abstand (a) angeordneter Scherenarme (17, 18) am Laufwagen (2) angelenkt ist, wobei zwischen den Gelenkstellen (19, 21 ; 20, 22) wenigstens eines Scherenarmes (17, 18) Antriebsmittel (23, 24) zur Erzielung der Schwenkbewegung sowie einer Änderung des Abstandes (b) zwischen Träger (6) und Laufwagen (2) angeordnet sind.

4. Gerät nach einem der Ansprüche 1 bis 3, bei dem zur Änderung des Abstandes (b) zwischen Träger (6) und Laufwagen (2) ein Teleskoparm (26) vorgesehen ist, dessen eines Ende mittels der Schwenkachse (25) am Lagerteil (7, 100) angelenkt ist und dessen anderes Ende am Laufwagen (2) starr befestigt ist.

5. Gerät nach Anspruch 3, bei dem zwischen den Gelenkstellen (19 bis 22) der Scherenarme (17, 18) je ein Spindelantrieb (23, 24) vorgesehen ist.

6. Gerät nach einem der Ansprüche 1 bis 5, bei dem der Drehring (6) einseitig an in einem abgewinkelten Lagerteil (7, 33) gefaßt ist, welches an seinem unteren Schenkelabschnitt zwei Führungsrollen (31, 32) enthält, an welchen der Ring mit einem an seiner Unterseite angeordneten Laufprofil (14) aufliegt und welches an seinem oberen Schenkelabschnitt eine Antriebsrolle (30) enthält, die mit den beiden Führungsrollen (31, 32) eine Dreieck-Auflage bilden und daß der Drehring (6) an seiner Ober- und Unterseite über wenigstens 240° seines Umfanges ein Laufprofil (14) aufweist, auf dem sich die am Lagerteil (7) angeordneten Führungsrollen (30 bis 32) abwälzen.

7. Gerät nach Anspruch 6, bei dem eine der Führungsrollen (30 bis 32) als mit einem Antriebsmotor (34) gekuppelte Antriebsrolle (30) ausgebildet ist, die aus zwei eine Lauffläche (46) für das Laufprofil bildenden Hälften (30a, 30b) besteht, von denen die eine (30a) fest auf einer mit dem Antriebsmotor (34) gekuppelten Achse (45) befestigt ist und die andere (30b) auf der Achse (34) so angeordnet ist, daß der die Lauffläche (46) aufweisende Teil gegenüber der anderen Rollenhälfte (30a) frei drehbar ist.

8. Gerät nach Anspruch 6, bei dem als Führungsrollen zwei auf einer gemeinsamen Achse (35) nebeneinander angeordnete Kugellager (36, 37) vorgesehen sind, deren Außenringe gegeneinander frei drehbar sind und eine Lauffläche (38) für das am Drehring (6) angeordnete Laufprofil (14) aufweisen und zumindest eine der Führungsrollen (30 bis 32) mittels einer Exzenterachse (35) im Lagerteil (7, 33) gelagert ist.

9. Gerät nach einem der Ansprüche 1 bis 8, bei dem der Drehring (6) in einem konzentrisch dazu angeordneten ringförmigen Lagerteil (100) drehbar gelagert ist.

10. Gerät nach Anspruch 9, bei dem der Drehring (6) mittels eines Bandantriebes (103, 104) angetrieben wird.

11. Gerät nach einem der Ansprüche 2 bis 10, bei dem der Drehring (6) aus einem umschäumten oder mit Schaumstoff umkleideten, ein Laufprofil (14, 102) für den Antrieb enthaltenden Träger (40), 47, 51) gebildet ist.

12. Gerät nach Anspruch 11, bei dem der Träger (40) aus zwei miteinander verschweißten Hälften (40a, 40b) aus gerolltem Blech gebildet ist.

13. Gerät nach Anspruch 12, bei dem Ober- und Unterseite der Hälften (40a, 40b) eine U-förmige Verbreiterung (43) bilden, in der das Laufprofil (14) und Kabelführungsrillen (15) bildende Teile (44) eingesetzt sind.

14. Gerät nach Anspruch 13, bei dem in die Verbreiterung zwei auf Abstand gehaltene, das Laufprofil bildende Ringe (55) eingelegt sind, auf denen die Antriebsrolle (30, 58) und die Führungsrollen (31, 32) mit jeweils Außenlaufflächen (56, 57) abrollen.

15. Gerät nach Anspruch 6, bei dem das Laufprofil durch einen einzigen, in einer Nut eines I-Trägers eingelegten Ring gebildet ist.

## Claims

1. A dental x-ray diagnostic apparatus for creating panoramic tomograms of the jaw of a patient, comprising a rotary unit (3), with a carrier (6), arranged on a travelling carriage (2) of a stand (1) on which a radiation source (4) and diametrically opposed thereto a holder (5) for a film cassette (11) accommodating an x-ray film, are able to be held so that they can be adjusted, such that emanating from the radiation source, rays penetrating the object (jaw) strike x-ray film substantially perpendicularly, comprising furthermore a first adjusting device (12), with which the film cassette (11) is able to be adjusted relative to the radiation source (4) and a second adjusting device, with which the carrier (6) is able to be adjusted in an orbital curve corresponding to the dental arch, wherein the second adjusting device contains first adjusting means (30 to 34) with which the carrier (6) can be rotated around a first vertical axis (27), second adjusting means (17 to 25), with which the carrier (6) during rotational motion around the first axis can execute a swivelling motion around a swivelling axis (25), transverse to the axis of symmetry of the object, and third adjusting means (17 to 26), with which the distance (b) of the carrier (6) to the travelling carriage (2) is able to be adjusted, wherein the swivelling radius (R) and the deflection (c) of the swivelling motion, which are able to be achieved with the second adjusting means, are selected so that with motion of the rotary unit a perpendicular penetration direction is achieved through the object which constant distance (d) from the object and film.

2. An apparatus according to claim 1, in which the carrier is formed preferably as a closed rotary ring (6) which is rotatably mounted on a bearing point (7, 100), connected to the travelling carriage (2) and accomodating the swivelling axis (25), wherein the central point (27) of the rotary ring (6) forms the rotary axis for the first adjusting means (30 to 34).

3. An apparatus according to claim 2, in which the bearing part (7, 100) is hinged to the travelling carriage (2) by means of two scissor arms (17, 18) arranged on both sides of the swivelling axis (25) at a distance (a), wherein between the joint points (19, 21 ; 20, 22) of at least one scissor arm (17, 18) there are arranged drive means (23, 24) for achieving the swivelling motion as well as an alteration of the distance (b) between the carrier (6) and travelling carriage (2).

4. An apparatus according to one of claims 1 to 3, in which there is provided for altering the distance (b) between the carrier (6) and travelling carriage (2) a telescopic arm (26), the one end of which is able to be hinged to the bearing point (7, 100) by means of the swivelling axis (25), and the other end of which is rigidly secured to the travelling carriage (2).

5. An apparatus according to claim 3, in which there is provided in each case between the hinge points (19 to 22) of the scissor arms (17, 18) a spindle drive (23, 24).

6. An apparatus according to one of claims 1 to 5, in which the rotary ring (6) is gripped on one side in a bent bearing part (7, 33), which comprises on its lower side section two guide rollers (31, 32), at which the ring lies with a runner profile (14) arranged on its lower side and which contains on its upper side section a drive roller (30), which forms together with both guide rollers (31, 32) a triangular bearing and that [sic] the rotary ring (6) has on its upper and lower side along at least 240° of its periphery a runner profile (14), on which the guide rollers (30 to 32) arranged on the bearing part (7) roll.

7. An apparatus according to claim 6, in which one of the guide rollers (30 to 32) is constructed as a drive roller consists of two halves (30a, 30b) forming a running face (46) for the running profile, one (30a) of which is secured firmly to an axis (45) coupled to the drive motor (34), and the other (30b) is arranged on the axis (34), so that the part having the running face (46) is freely rotatably relative to the other roller half (30a).

8. An apparatus according to claim 6, in which two ball bearings (36, 37) arranged next to each other on a common axis (35) are provided as guide rollers, the outer rings of which are freely rotatably relative to each other and have a running face (38) for the running profile (14) arranged on the rotary ring (6), and at least one of the guide rollers (30 to 32) is mounted by means of an eccentric axis (35) in the bearing part (7, 33).

9. An apparatus according to one of claims 1 to 8, in which the rotary ring (6) is rotatably mounted in an annular bearing part (100) arranged concentrically thereto.

10. An apparatus according to claim 9, in which the rotary ring (6) is driven by means of a tape drive (103, 104).

11. An apparatus according to one of claims 2 to 10, in which the rotary ring (6) is formed from a carrier (40, 47, 51), coated or covered by foam, containing a runner profile (14, 102) for the drive.

12. An apparatus according to claim 11, in which the carrier (40) is formed from two halves (40a, 40b) of rolled sheet metal welded together.

13. An apparatus according to claim 12, in which the upper and lower side of the halves (40a, 40b) form a U-shaped widening (43), in which the parts (44) forming the runner profile (14) and the cable guide grooves (15) are placed.

14. An apparatus according to claim 13, in which two rings (55), held at a separation, and forming the running profile are inserted into the widening, on which rings the drive roller (30, 58) and the guide rollers (31, 32) roll with respective outer running faces (56, 57).

15. An apparatus according to claim 6, in which the runner profile is formed by a single ring inserted in a groove of an I-carrier.

## Revendications

1. Appareil de radiodiagnostic dentaire pour réaliser des tomographies panoramiques de la mâchoire d'un patient, comportant une unité rotative (3) montée sur un chariot coulissant (2) d'un statif (1) et possédant un support (6), sur lequel une source de rayonnement (4) et, en position diamétralement opposée, un support (5) pour une cassette à film (11) logeant un film radiographique sont fixés de manière à être déplaçables, de sorte que des rayons, qui partent de la source de rayonnement et traversent l'objet (mâchoire), tombent sensiblement perpendiculairement sur le film radiographique, et comportant en outre un premier dispositif de déplacement (12), à l'aide duquel la cassette à film (11) est déplaçable par rapport à la source de rayonnement (4), et un second dispositif de déplacement, au moyen duquel le support (6) peut être déplacé suivant une courbe de déplacement correspondant à l'arc dentaire, et dans lequel le second dispositif de déplacement comporte des premiers moyens de déplacement (30 à 34), à l'aide desquels le support (6) peut être entraîné en rotation autour d'un axe vertical (27), des seconds moyens de déplacement (17 à 25), à l'aide desquels le support (6) peut exécuter, autour d'un axe de pivotement (25), un mouvement de pivotement transversalement par rapport à l'axe de symétrie de l'objet, pendant le mouvement de rotation autour du premier axe, et des troisièmes moyens de déplacement (17 à 26), à l'aide desquels la distance (b) entre le support (6) et le chariot coulissant (2) peut être réglée, le rayon de pivotement (R) et l'amplitude (c) du mouvement de pivotement, que l'on peut obtenir avec les seconds moyens de déplacement, étant choisis de manière que, lors du déplacement de l'unité rotative, on obtient en permanence une direction perpendiculaire d'irradiation de l'objet, et ce avec une distance (d) constante entre l'objet et le film.

2. Appareil suivant la revendication 1, dans lequel le support est agencé sous la forme d'un anneau tournant (6) de préférence fermé, qui est maintenu de manière à pouvoir tourner sur un élément de support (7, 100) raccordé au chariot coulissant (2) et logeant l'axe de pivotement (25), le centre (27) de l'anneau rotatif (6) constituant l'axe de rotation pour les premiers moyens de déplacement (30 à 34).

3. Appareil suivant la revendication 2, dans lequel l'élément de support (7, 100) est articulé sur le chariot coulissant (2) au moyen de deux branches de ciseaux (17, 18) disposées des deux côtés de l'axe de pivotement (25) à une distance (a), des moyens d'entraînement (23, 24) permettant d'obtenir le mouvement de pivotement ainsi qu'une modification de la distance (b) entre le support (6) et le chariot (2) étant disposés entre les points d'articulation (19, 21 ; 20, 22) d'au moins une branche de ciseaux (17, 18).

4. Appareil suivant l'une des revendications 1 à 3, dans lequel pour modifier la distance (b) entre le support (6) et le chariot (802), il est prévu un bras télescopique (26), dont une extrémité est articulée au moyen de l'axe de pivotement (25) sur l'élément de support (7, 100) et dont l'autre extrémité est fixée rigidement au chariot (2).

5. Appareil suivant la revendication 3, dans lequel respectivement un dispositif d'entraînement à broches (23, 24) est prévu entre les articulations (19 à 22) des branches de ciseaux (17, 18).

6. Appareil suivant l'une des revendications 1 à 5, dans lequel l'anneau rotatif (6) est retenu unilatéralement dans un élément de support coudé (7, 33), qui comporte, au niveau de sa branche inférieure, des galets de guidage (31, 32) contre lesquels l'anneau s'applique par un profil de roulement (14) présent au niveau de sa face inférieure et qui contient, sur sa branche supérieure, un galet d'entraînement (30), qui forme, avec les deux galets de guidage (31, 32) un support en triangle, et que l'anneau rotatif (6) possède, au niveau de sa face supérieure et de sa face inférieure et sur au moins 240° de son pourtour, un profil de roulement (14), sur lequel roulent les galets de guidage (30 à 32) montés sur l'élément de support (7).

7. Appareil suivant la revendication 6, dans lequel l'un des galets de guidage (30 à 32) est agencé sous la forme d'un galet d'entraînement (30), accouplé à un moteur d'entraînement (34) et formé de deux moitiés (30a, 30b) constituant une surface de roulement (46) pour le profil de roulement et dont l'un (30a) est fixé fermement à un axe (45) accouplé au moteur d'entraînement (34) et dont l'autre (30b) est disposé sur l'axe (34) de telle sorte que la partie portant la surface de roulement (46) peut tourner librement par rapport à l'autre moitié (30a) du galet d'entraînement.

8. Appareil suivant la revendication 6, dans lequel il est prévu, comme galets de guidage, deux roulements à billes (36, 37), qui sont disposés côte-à-côte sur un axe commun (35) et dont les pistes extérieures peuvent tourner librement l'une par rapport à l'autre et possèdent une surface de roulement (38) pour le profil de roulement (14) situé sur l'anneau rotatif (6), au moins l'un des galets de guidage (30 à 32) étant supporté au moyen d'un axe excentré (35) dans l'élément de support (7, 33).

9. Appareil selon l'une des revendications 1 à 8, dans lequel l'anneau rotatif (6) est monté rotatif dans un élément de support annulaire (100) disposé concentriquement par rapport à cet anneau.

10. Appareil suivant la revendication 9, dans lequel l'anneau rotatif (6) est entraîné à l'aide d'un dispositif d'entraînement en forme de bande (103, 104).

11. Appareil suivant l'une des revendications 2 à 10, dans lequel l'anneau rotatif (6) est formé par un support (40, 47, 51) entouré de mousse ou revêtu d'un matériau mousse et comportant un profil de roulement (14, 102) pour le dispositif d'entraînement.

12. Appareil suivant la revendication 11, dans lequel le support (40) est formé de deux moitiés (40a, 40b) soudées entre elles et constituées par une tôle laminée.

13. Appareil suivant la revendication 12, dans lequel la face supérieure et la face inférieure des moitiés (40a, 40b) forment une partie élargie en forme de U (43), dans laquelle sont insérées des parties (44) formant le profil de roulement (14) et des rainures (15) de guidage d'un câble.

14. Appareil suivant la revendication 13, dans lequel deux anneaux (55), qui sont maintenus à distance, et constituent le profil de roulement, et sur lesquels les galets d'entraînement (30, 58) et les galets de guidage (31, 32) roulent au moyen de leurs surfaces extérieures respectives de roulement (56, 57), sont insérés dans la partie élargie.

15. Appareil suivant la revendication 6, dans lequel le profil de roulement est formé par un seul anneau inséré dans une rainure d'un support en forme de I.

FIG 1

FIG 9

FIG 10

FIG 2

FIG 3

FIG 4

FIG 5

11

FIG 6

FIG 7

FIG 8